# EUROPEAN PATENT SPECIFICATION

(11) **EP 3 273 930 B1**
(45) Date of publication and mention of the grant of the patent: **24.08.2022**
(21) Application number: 15780936.9
(22) Date of filing: 03.09.2015
(51) Int. Cl.: A61K 8/24, A61K 8/73, A61Q 17/04, A61K 8/11, A61K 8/27

(54) **COMPOSITE CONTAINING CHITOSAN AND ZINC OXIDE NANOPARTICLES AND A PROCESS FOR ITS PREPARATION**
VERBUNDSTOFF MIT CHITOSAN- UND ZINKOXIDNANOPARTIKELN UND VERFAHREN ZU DESSEN HERSTELLUNG
COMPOSITE CONTENANT DU CHITOSANE ET DES NANOPARTICULES D'OXYDE DE ZINC, ET SON PROCÉDÉ DE PRÉPARATION

(30) Priority: 27.03.2015 PL 41174215
(43) Date of publication of application: 31.01.2018
(73) Proprietor: Uniwersytet Jagiellonski, 31-007 Krakow (PL)
(72) Inventor: MACYK, Wojciech, PL-32-060 Liszki (PL); REGIEL-FUTYRA, Anna, PL-30-409 Kraków (PL); STOCHEL, Grazyna, PL-31-235 Kraków (PL)
(74) Representative: Witek, Rafal
(86) International application number: PCT/IB2015/056709
(87) International publication number: WO 2016/156939

(56) References cited:
- EP-A1- 2 848 247
- WO-A1-2008/126971
- KR-A- 20140 080 467

## Description

Commercially available sun creams/topical sunscreens comprise two classes: inorganic and organic. The main components of the former class, constituting a kind of barrier to UV radiation, are typically nano- and microcrystalline forms of semiconductors, such as titanium dioxide (TiO₂) and zinc oxide (ZnO). Their task is to absorb and reflect and scatter the radiation of the ultraviolet range - UV-A (320-400 nm) and UV-B (290-320 nm)¹. These creams are transparent to visible light, barely adhesive and easily distribute on the skin². It is further proposed that the ease with which they are absorbed by the epidermis, without skin irritation, constitutes an additional advantage^{3,4}.

In addition to many advantages of this type of cream, there is a considerable risk arising from their use. The main concern relates to the toxicity of nanoparticles of the used photocatalysts, which is mainly the result of: (1) their size, (2) the possibility to circumvent immune defense mechanisms and (3) a tendency to form complexes with proteins. However, the most important concern related to the application of nanometric oxides as main cream components is generation of reactive oxygen species (ROS). ROS are produced by a photocatalytic process on the surface of semiconductors (including metal oxides), based on an interfacial transfer of photogenerated charges involving oxygen molecules or water or a transfer of energy to an oxygen molecule. The resulting strongly oxidizing hydroxyl (OH•) and superoxide (O₂•-) radicals, hydrogen peroxide (H₂O₂) or singlet oxygen (¹O₂) are known as cytotoxic and mutagenic agents⁵⁻⁷. They may lead to oxidation of organic components of the cream, photodamage of proteins, lipids and DNA⁸⁻¹⁰.

In order to minimize photocatalytic reactions involving TiO₂ and ZnO, it is recommended to use deactivated forms of semiconductors, which are obtained by chemical modification of their surface or application of an additional component of creams - antioxidants (e.g. β-carotene, ascorbic acid, glutathione, etc.)¹¹. Another described example is nano- and microparticles of TiO₂ or ZnO coated with SiO₂, Al₂O₃, for which a reduced activity induced by the absorbed light was demonstrated^{8,10}. An interesting and promising example is also the use of polymer films with natural antioxidants¹². However, not every modification of a semiconductor surface ultimately leads to a reduction in photocatalytic activity¹⁰. Therefore, an important task is to search for modern composite materials which would constitute a barrier to UV radiation, while minimizing the toxic effects of photocatalytic reactions.

Another important problem of the use of nanoparticles of semiconductors as sunscreens relates to their dimensions (<100 nm), enabling free penetration through the stratum corneum of the skin and, consequently, penetration to deeper layers of the skin. The European Scientific Committee on Consumer Products, based on the analysis of a number of studies in this field, emphasized the lack of sufficient data to assess the safety of topical application of nanoparticles and the possible side effects¹³. Literature indicates that nanoparticles administered topically to the skin ultimately penetrate to the lymph nodes¹⁴. Studies demonstrated that endothelial cells have a high capacity to internalize the nanoparticles, which raised further concern about potential inflammation and cytotoxic activity¹⁵. While the debate on the safety of the use of nanoparticles continues, minimization of the side effects of the use of sunscreen preparations is needed.

Korean patent application KR 2014 0080467 A discloses in example 2 the manufacture of chitosan nanoparticles containing zinc oxide via inotropic gelation with tripolyphosphate. The particles obtained have a size of 10-500 nm.

International patent application WO 2008/126971 A1 discloses UV-blocking material containing polymer nanoparticles and methods for their preparation. In example 2, the preparation of zinc oxide-containing chitosan nanoparticle is disclosed.

The aim of the present invention is to provide a new biocompatible material which would have the ability to absorb UV-A/UV-B radiation and is suitable for the manufacture of cosmetic products, particularly sun creams.

The present invention relates to a process for the preparation of a colloid containing a composite, wherein:
a) a suspension of grains of zinc oxide (ZnO) in a solution of chitosan (CS) is obtained,
b) the resulting suspension is mixed with a solution of sodium tripolyphosphate (TPP), and a reaction of ionotropic crosslinking is conducted,
c) the resulting mixture is treated with ultrasound, and the colloid containing the composite is obtained.

Preferably, in step a) the grains of zinc oxide have a size not greater than 100 nm.

Preferably, the suspension obtained in step a) comprises equal concentrations of zinc oxide and chitosan.

Preferably, the mixture obtained in step b) comprises sodium tripolyphosphate at a concentration twice as high as the concentration of chitosan.

Further, the invention relates to a colloid containing a composite comprising aggregates consisting of grains of zinc oxide trapped in chitosan ionotropically crosslinked with tripolyphosphate, wherein the aggregates have a size not greater than 100 micrometers.

In disclosed embodiments, it has at least one of the following features:
- infrared absorption spectrum exhibits a widened band at ~3290 cm⁻¹, observed for pure chitosan and its shift towards a lower wavenumber, in particular ~3276 cm⁻¹ or ~3259 cm⁻¹,
- disappearance of photocurrents measured in a three-electrode system (electrode coated with a described material, Pt, Ag/AgCl) in the wavelength range 330-450 nm,
- loss of photocatalytic activity, especially including the inability to decompose azure B and photodegradation of bovine serum albumin,
- loss of the ability to generate singlet oxygen upon exposure to light, measured as the ability to oxidize α-terpinene to ascaridole,
- bactericidal activity, particularly against bacteria of the *Staphylococcus aureus* or *Escherichia coli* species,
- lack of cytotoxic activity against human keratinocytes measured in the MTT assay.

The presented materials are nanocomposites based on nanoparticles of zinc oxide (ZnO) superficially modified with a biocompatible polymer - chitosan (CS). Excellent absorption of UV-A/UV-B radiation in combination with a low refractive index, chemical stability and low toxicity result in that ZnO nanoparticles are widely used as ingredients of sun creams^{16,17}. In the study that led to the present invention, commercially available nanoparticles of zinc oxide of grain size ≤50 nm and ≤100 nm and chitosan of a mean molecular weight ~1300 kDa were applied. The technique of ionotropic gelation with sodium tripolyphosphate as a crosslinking agent was used to obtain ZnO nanoparticles superficially modified with CS (CS@ZnO). Surprisingly, in the materials obtained according to the invention, a lack of photocatalytic activity towards the dye (i.e. azure B) sensitive to various ROS forms and oxidants and the model biological molecule (bovine serum albumin) was found. Further, no generation of photocurrents within a wide range of applied voltage and wavelength used in irradiation was confirmed, which indicated a lack of interfacial processes of electron transfer between the material and the environment. Despite superficial modification, CS@ZnO materials absorb the light from the UV range (<400 nm). The presented materials also do not show activity for the generation of singlet oxygen. Preliminary biological tests confirmed their bactericidal activity against antibiotic resistant Gram-(+) and Gram-(-) bacterial strains (*Staphylococcus aureus, Escherichia coli*), while demonstrating no cytotoxic effects against human keratinocytes. The presented properties of CS@ZnO allow for the management of the risks described in the introduction, which are associated with the use of semiconductor nanoparticles as active ingredients of sunscreen preparations. The said polymeric coating, which allows for the absorption of UV radiation, provides protection for particles potentially susceptible to photodegradation.

In literature, there are previous reports on ZnO-chitosan composites. Numerous studies describe materials ZnO/CS, for which antibacterial and antifungal activity was demonstrated¹⁷. Li et al. presented CS-nano-ZnO membranes exhibiting activity against Gram-(-) *(Bacillus subtilis, Eschierichia coli*) and Gram-(+) (*Staphylococcus aureus*) bacterial strains, but there is no information concerning their photoactivity¹⁸. Similar bactericidal activity against *Vibrio parahaemolyticus* and *Bacillus lechiniformis* was shown by Vaseeharan et al.¹⁹. In both cases, the absorption properties (<400 nm) of materials modified with chitosan were maintained, which was also confirmed in the present study. Further, the study described by AbdElhady et al. relates to cotton fibers coated with a CS/ZnO-NPs composite to protect it against UV radiation and to introduce antibacterial activity²⁰. Farouk et al. also demonstrated the antibacterial potential of cotton fibers modified with chitosan/ZnO-NPs sol²¹. In contrast to the described examples, Sanoop et al. described Zn/biopolymer nanocomposite coatings (including chitosan), applied to modify polyurethane/cotton foams, showing photoactivity in UV²².

Based on the research studies described above, it is worth emphasizing that the materials of the present application, in addition to their widely described antimicrobial activity, solve the problem of material photoactivity within the UV range, resulting in the formation of reactive oxygen species and subsequent side effects.

In order to better reveal the essence of the present invention, the description was supplemented with the following figures:
Figure 1: a scheme of superficial modification of zinc nanoparticles (ZnO-NP) with selected polymer-chitosan;
Figure 2: powder diffraction patterns obtained for ZnO50 (A) and ZnO100 (B);
Figure 3: SEM images of the unmodified materials ZnO50 (A) and ZnO100 (D); zinc oxide materials with chitosan without crosslinking ZnO50-CS (B) and ZnO-CS (E) and modified materials CS@ZnO50 (C) and CS@ZnO100 (F);
Figure 4: infrared absorption spectra of: oxides ZnO50 (A) and ZnO (B) and chitosan (CS) and materials with polymer without crosslinking (-CS) and with crosslinked polymer (CS@);
Figure 5A-B: analysis of the elemental composition of zinc oxide ZnO50 (A) and ZnO100 (B);
Figure 5C: photocurrents registered for the materials based on ZnO50 and ZnO100; the height of peaks on the graphs, related to the opening and closing of the shutter, is a measure of the generated photocurrents;
Figure 6: reflectance spectra (DRS) of zinc oxide ZnO50 and ZnO100;
Figure 7: photodegradation of azure B in the presence of zinc oxide ZnO50 (A) and zinc oxide ZnO50 modified with chitosan (B) (A/A₀ - the ratio of the substrate concentration during the measurement to the initial concentration);
Figure 8: photodegradation of azure B in the presence of zinc oxide ZnO100 (A) and zinc oxide ZnO modified with chitosan (B);
Figure 9: photodegradation of bovine serum albumin (BSA) in the presence of zinc oxide ZnO50 and CS@ZnO50 (A) and zinc oxide ZnO100 and CS@ZnO100 (B);
Figure 10: results of antibacterial tests (CFU - Colony Forming Units) against the Gram-(+) strain of *Staphylococcus aureus* for ZnO50/CS@ZnO50 (A) and ZnO100/CS@ZnO100 (B);
Figure 11: results of antibacterial tests (CFU - Colony Forming Units) against the Gram-(-) strain of *Escherichia coli* for ZnO50/CS@ZnO50 (A) and ZnO100/CS@ZnO100 (B);
Figure 12: results of cytotoxicity tests against human keratinocytes.
Figures 13-15: images obtained using a scanning electron microscope allowed for analysis of the morphology of the produced materials. SEM also allows for the estimation of the size of aggregates according to the invention.

Further, the present description has been supplemented with the following embodiments. They should not be identified with the full scope of the above-defined invention.

### Example 1. Production of ZnO nanoparticles superficially modified with chitosan.

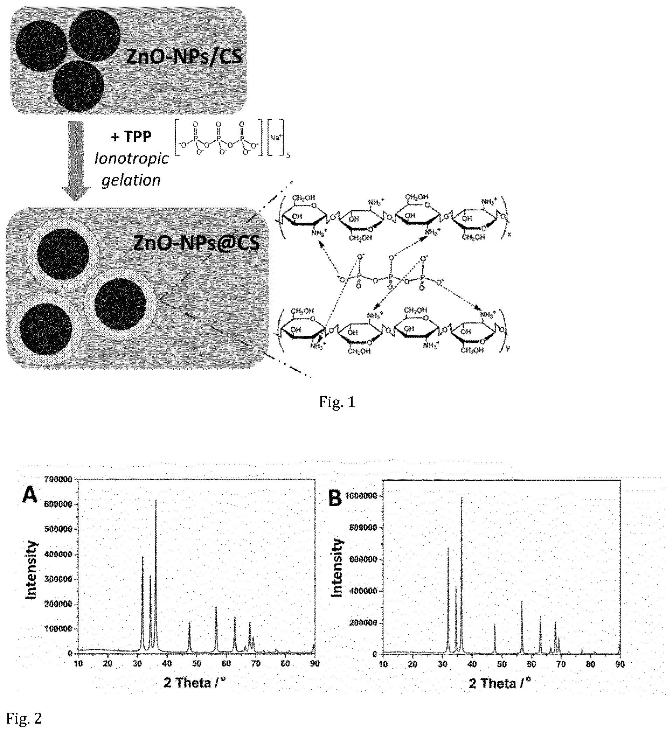

In Figure 1, a scheme of surface modification of zinc monoparticles with the selected polymer - chitosan was presented.

Commercially available samples of semiconductors - zinc oxide nanoparticles sized up to 50 nm (ZnO50) and up to 100 nm (ZnO100) - were used for the study. The materials were subjected to preliminary physicochemical tests and then to surface modification with the selected polymer, i.e. chitosan (CS). For this purpose, microencapsulation by ionotropic gelation between the polycationic polymer (pH≈4) and the selected anionic crosslinking agent - sodium tripolyphosphate - was applied. A suspension of ZnO nanoparticles at a concentration of 1 g/L was prepared in a solution of chitosan (1g/L). In the subsequent step, a crosslinking agent at a concentration of 2 g/L (at a volume ratio of 1:1) was added dropwise while stirring the suspension. 15 minutes after TPP addition, the samples were subjected to ultrasound (30 minutes, ultrasonic bath). This process leads to the formation of a colloidal system of semiconductor nanoparticles coated with superficially crosslinked polymer, the properties of which, such as size, zeta potential or stability, depend on the applied crosslinking conditions. The composite can be easily separated from the resulting colloid e.g. by centrifugation or filtration.

Further, it was established that the size of the resulting aggregates is also affected by the size of the applied chitosan particles. The mean molecular weight of chitosan was determined by measuring the viscosity numbers of diluted polymer solutions. Molar mass was calculated based on the limit value of the viscosity number using the semi-empirical equation of Mark-Houwink. Viscosimetric measurements were performed using an Ubbelohde-type viscometer (Visco System AVS 350, Schott-Gerate, Germany). A chitosan solution at a concentration of 1% (w/v) in 0.8% (v/v) acetic acid was purified by precipitation with sodium hydroxide, filtration and washing with water. The purified sample was dried at 65°C. Solutions at concentrations in the range of 0.1-0.5 mg/mL in 0.1 M acetic acid/0.2 M sodium chloride were prepared from a purified sample of polymer. Measurements for each concentration were performed five times at a temperature of 25 ± 0.1°C. The determined mean molecular weight of the polymer was 1278 ± 8 kDa1. The viscosity range specified by the manufacturer for the applied chitosan was 200-800 cP (1 wt. % in 1% acetic acid (25°C)).

A wide physicochemical and photochemical analysis and preliminary biological tests were performed for the initial (unmodified ZnO) and superficially modified samples, according to the invention.

### Example 2. Properties of the material according to the invention.

### Measurements of dynamic light scattering (DLS)

In order to determine the hydrodynamic size of ZnO and CS@ZnO nanoparticles, measurements of dynamic light scattering were performed. The results of DLS and polydispersity index (Pdl) measurements are summarized in Table 1.

**Table 1. Summary of test results using the Zeta-Sizer.**

| Sample | Size [nm] | PdI |
|---|---|---|
| ZnO50 | 167 ± 1 | 0.27 ± 0.02 |
| ZnO50@CS | 233 ± 2 | 0.4 ± 0.01 |
| ZnO100 | 288 ± 2 | 0.23 ± 0.01 |
| ZnO100@CS | 342 ± 14 | 0.17 ± 0.01 |

For both forms of ZnO, an increase in hydrodynamic diameter resulting from the modification with chitosan was observed. This increase testifies to the increase of polymer coating on the surface of a semiconductor.

### Crystal structure of semiconductors (X-Ray Powder Diffraction, XRD)

XRD measurements were performed to determine the crystal structure and phase composition of the test semiconductors. In Figure 2, powder diffraction patterns obtained for ZnO (A) and ZnO (B) were presented. Analysis of the diffraction patterns confirmed the polycrystalline structure of wurtzite and a hexagonal system of zinc oxide nanoparticles.

### Analysis of the morphology of the test materials

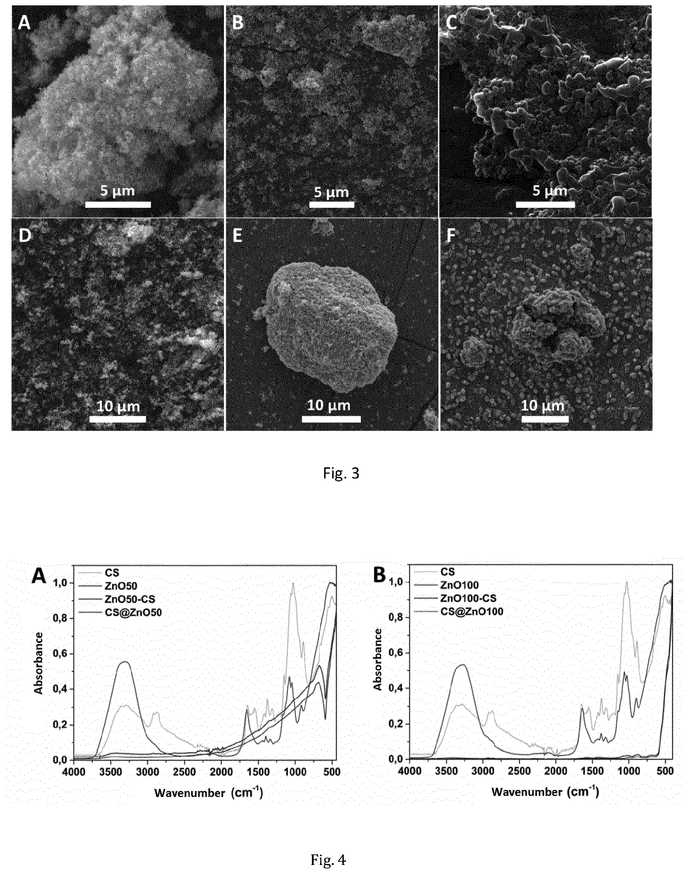

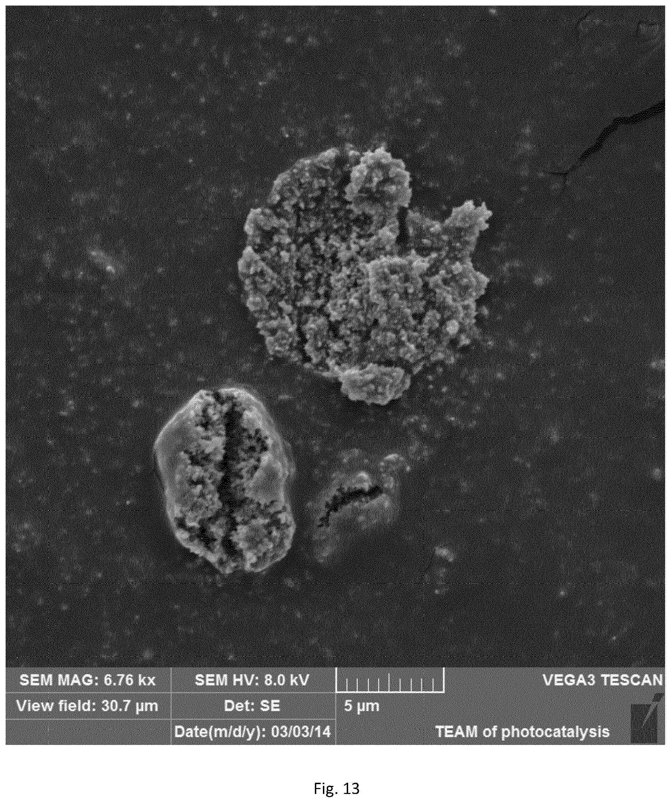

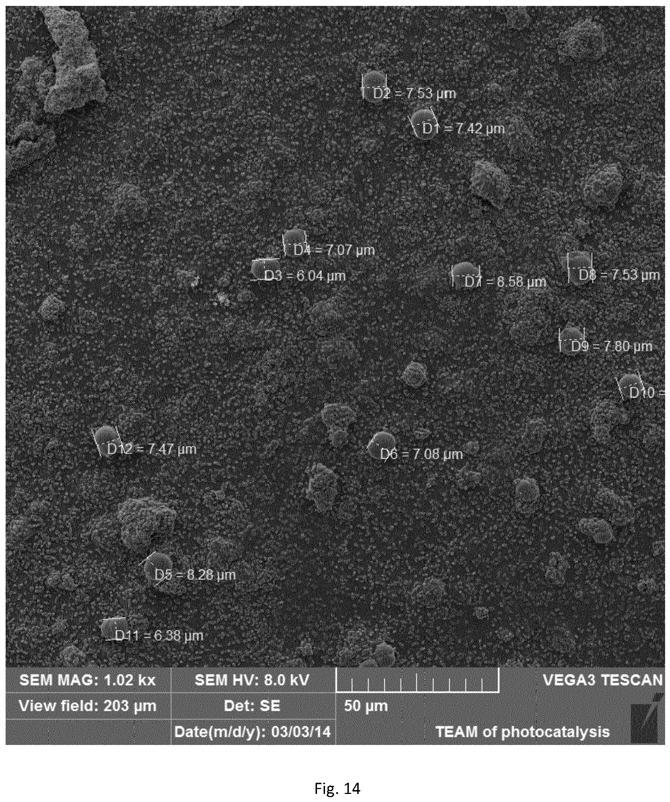

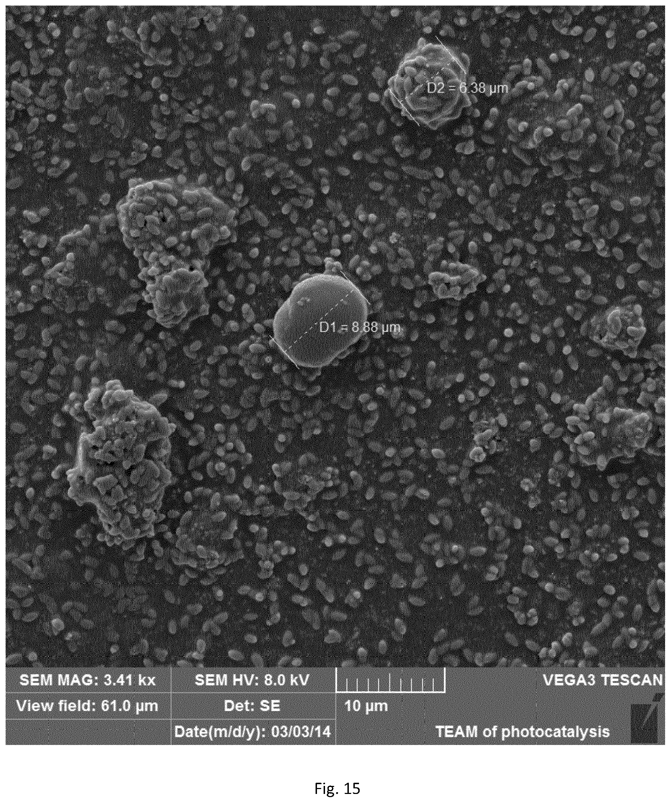

Images obtained using a scanning electron microscope allowed for analysis of the morphology of the produced materials. The unmodified zinc oxide 50 (Fig. 3A) and 100 (Fig. 3D) is characterized by very fine grains (size ≤ nm). Despite incubation of ZnO50 and ZnO100 in a chitosan solution, no significant impact of the polymer on the morphology of the samples is visible; they remained in the form of fine, sometimes glued grains (Fig. 3B, E). In contrast, materials modified with chitosan using ionotropic crosslinking show significant changes in morphology (Fig. 3C, F). The crosslinked polymer covers the aggregates of ZnO grains, which can be observed as oval and smooth shapes of glued grains. SEM also allows for the estimation of the size of aggregates according to the invention (see Fig. 13-15).

### Analysis of infrared absorption spectra

An absorption spectra of the materials allowed for analysis of the interaction of functional groups of chitosan with the surface of zinc oxide nanoparticles and for a confirmation of a significant impact of polymer crosslinking on the final form of the material. In order to demonstrate the presence of chitosan on the surface of ZnO nanoparticles, measurements for unmodified materials (ZnO), for materials with chitosan without crosslinking (ZnO-CS) and for composites CS@ZnO were performed. Further, a spectrum for a crosslinked polymer was presented (Fig. 4). The analysis of spectra of the materials without crosslinking of the polymer leads to a conclusion on the absence of chitosan in the sample. In turn, for the materials with the crosslinked polymer, significant changes in the absorption spectrum were observed. Primarily, bands derived from the polymer appeared, which confirms its presence on the ZnO surface. Furthermore, significant changes in the intensity and location of the bands derived from the functional groups of the polymer, resulting from the interaction with the ZnO nanoparticle surface, were shown.

The band of ~3290 cm⁻¹ for the pure polymer, resulting from stretching vibrations within the amino (-NH₂) and hydroxyl (-OH) groups for the crosslinked materials, is visibly widened and shifted towards a lower wavenumber, which indicates the interaction of these groups with ZnO (Salehi, R.; Arami, M.; Mahmoodi, N. M.; Bahrami, H.; Khorramfar, S. Colloids and Surfaces B: Biointerfaces 2010, 80, (1), 86-93.). For the materials CS@ZnO50 and CS@Zn0100, the maximum bandwidth is successively at ~3276 cm⁻¹ and ~3259 cm¹. Further, changes in the position and intensity of bands: amide I and amide II located at 1450, 1550 and 1650 cm⁴, were observed, which also confirms the interaction of amino groups with ZnO (AbdElhady, M. M. International Journal of Carbohydrate Chemistry 2012, 2012, 6.).

### EDS analysis

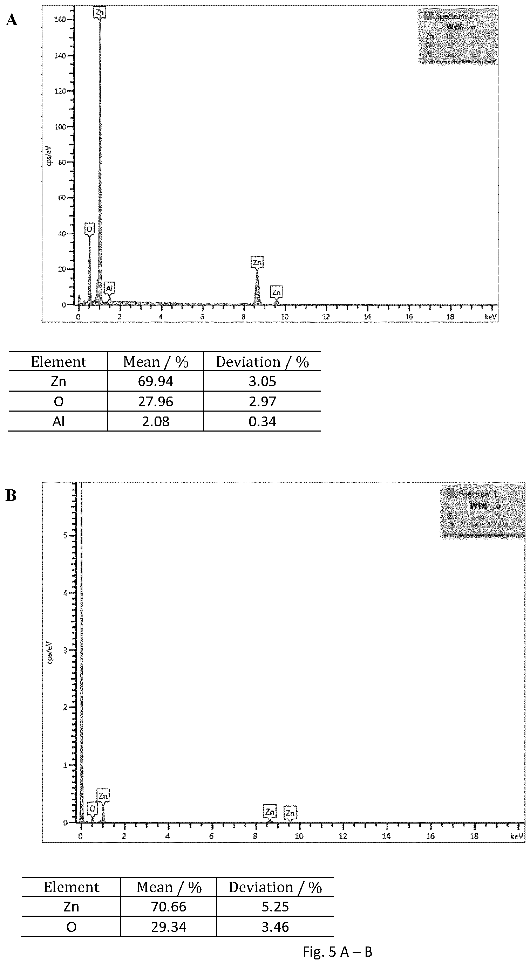

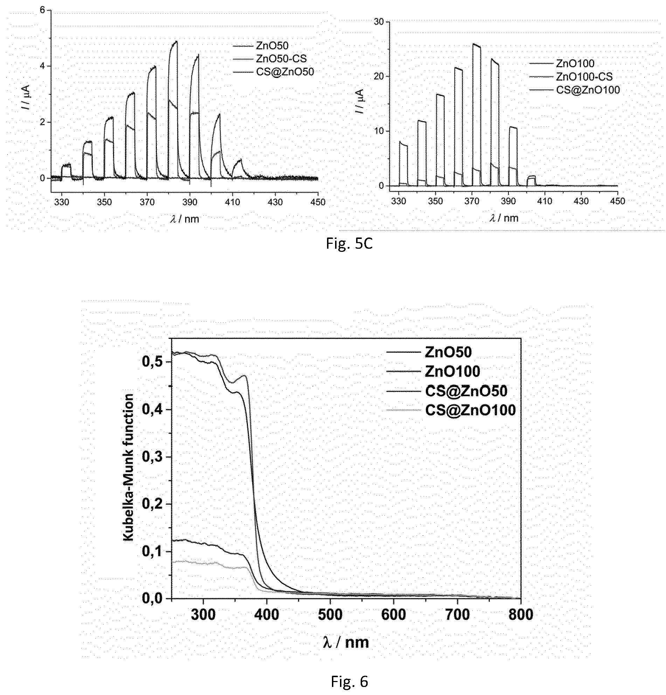

Measurements of Energy-Dispersive X-ray Spectroscopy (EDS) allowed for further analysis of the elemental composition of the study zinc oxide nanoparticles (Fig. 5). Figure 5 presents the results of the analysis of the elemental composition of zinc oxide ZnO50 (Fig. 5A) and ZnO100 (Fig. 5B).

### Photoelectrochemical tests

Photocurrent measurements were performed in a three-electrode system (working electrode, Pt, Ag/Ag/Cl). The working electrode was ITO film coated with the test material. Measurements were conducted in the wavelength range 330-450 nm at a potential of 0.2 V vs. Ag/AgCl for the materials without modification (ZnO), with non-crosslinked polymer (ZnO-CS) and with crosslinked polymer (CS@ZnO). Studies have shown a reduction in the values of the observed photocurrents for ZnO-CS materials and their total disappearance for the materials with the crosslinked polymer CS@ZnO (Fig. 5C), which also confirms complete isolation of the semiconductor from the environment by the crosslinked polymer and the impossibility of charge transfer between ZnO grains and the solution. This result demonstrates that the process of chitosan crosslinking is crucial for the complete quenching of photoactivity of the materials.

### Analysis of reflectance spectra of ZnO and CS@ZnO

Reflectance spectra (DRS) were made using a UV-3600 spectrophotometer provided by Shimadzu. The samples were triturated with BaSO₄, which was also a reference (at a weight ratio of approx. 1:30), in an agate mortar. The resulting spectra are shown below (Figure 6). In Figure 6, the reflectance spectra of zinc oxide ZnO50 and ZnO100 were presented. The modified materials preserved their absorptive properties <400 nm.

### Determination of photocatalytic activity

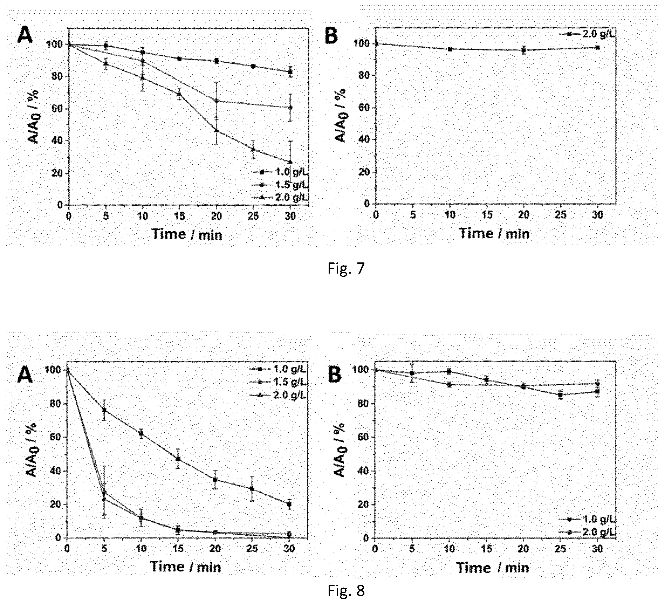

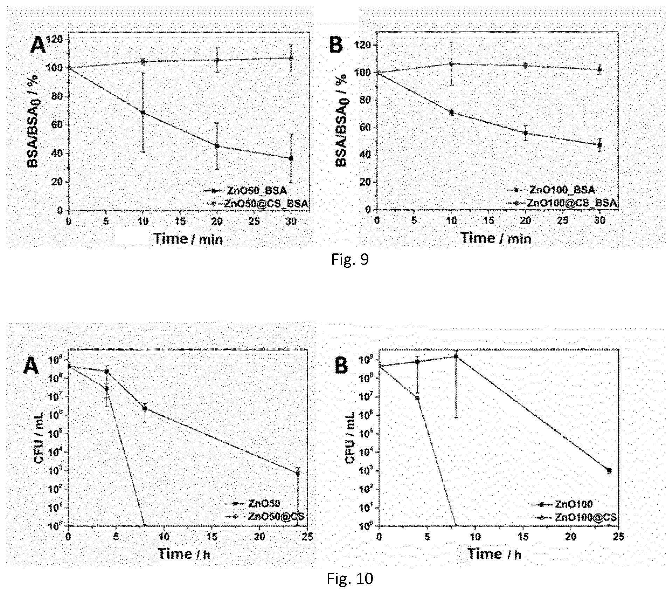

In a next step, photocatalytic tests for the initial and modified semiconductors were conducted using selected substrates or a dye - azure B (Figure 7, 8) - and a model biological molecule - bovine serum albumin (BSA, Figure 9). Photocatalytic activity was determined by monitoring photodegradation of the selected substrates.

ZnO and CS@ZnO suspensions in aqueous solutions of substrates were irradiated with an HBO-200 lamp (through a 10 cm water filter, using high-pass filters 320 and 495 nm, by blowing air). The samples were collected at specified time intervals and then subjected to analysis.

In the case of azure B decomposition, the collected samples were purified using Millipore syringe filters and subjected to UV-VIS measurements (Hewlett Packard HP8453). The progress of the photodegradation process was determined by the change in absorbance (corresponding to the concentration) of azure B (λ=650 nm). Photodegradation of bovine serum albumin was also determined by monitoring the changes in protein concentration using a densitometric electrophoretic analysis.

Figure 7 shows the photodegradation of azure B in the presence of ZnO50 (A) and CS@ZnO50 (B). While for ZnO50 (Figure 7A), a progressive degradation of the dye (dependent on the applied photocatalyst concentration) can be observed. The substrate concentration for the chitosan-modified material remained unchanged (Figure 7B).

Similar results were obtained for ZnO100 and CS@ZnO100 (Figure 8). Even for the lowest of the applied concentrations of ZnO100 (1 g/L), after 30 minutes, 80% of degradation was obtained, while at higher concentration, a total degradation of the substrate occurred (Figure 8A). Similarly as previously, the lack of photocatalytic activity of CS@ZnO100 (Figure 8B) was demonstrated.

The lack of photocatalytic activity of materials modified with chitosan (CS@ZnO50/100) was also confirmed for bovine serum albumin. In turn, for ZnO, a degradation of albumin at a level of 40-50% during 30 minutes irradiation was demonstrated (Figure 9A and B).

The presented measurement results indicate a significant impact of the applied superficial modification of the semiconductor on photocatalytic activity, and actually its disappearance.

### Studies on the ability to generate singlet oxygen

In order to determine the ability to generate singlet oxygen by the test semiconductors, a series of irradiation was performed in the presence of α-terpinene, which undergoes oxidation to ascaridole under the influence of ¹O₂. A suspension of the test materials (0.5 g/L) in a methanol solution of α-terpinene (10⁻³ M) was irradiated for one hour, while blowing with air (XBO xenon lamp 150 W, 10 cm CuSO₄ (0.1 M) filter, cut-off filter 320 nm). Samples collected every 15 minutes were analyzed by HPLC (author's method). No ability to generate singlet oxygen was found for the materials according to the invention.

### Antibacterial tests

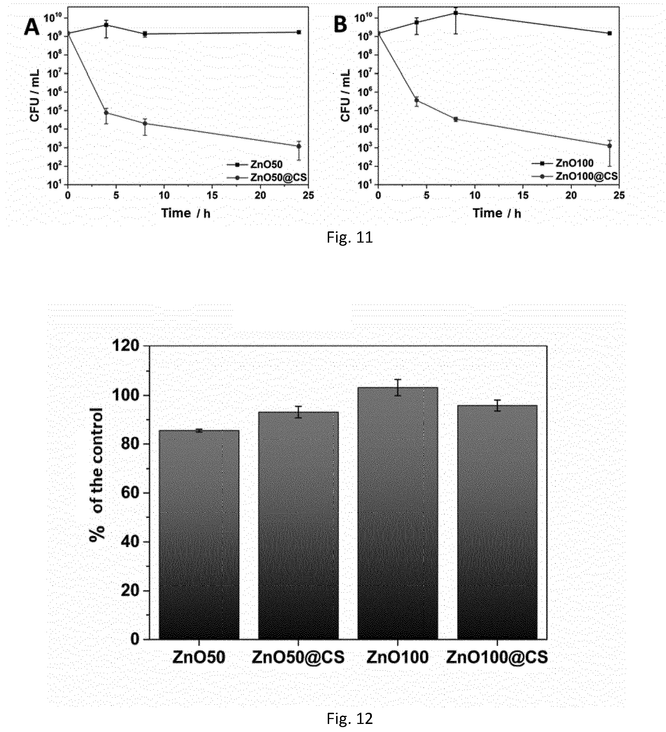

Tests of antibacterial activity against Gram-(+) *Staphylococcus aureus* and Gram-(-) *Escherichia coli* strains were performed for ZnO and CS@ZnO materials. The suspensions of materials of a concentration of 0.5 g/L were treated with a 24-hour suspension of bacteria (CFU~10⁹; final concentration after dilution of the bacterial suspension was 0.23 g/L). The samples were incubated at a simultaneous shaking for successive 4, 8 and 24 hours, and then smears on agar plates were made. After 24 hours, colonies were counted and presented graphically (Figure 10 and 11).

For the materials modified with chitosan, CS@ZnO, a complete bactericidal effect against *Staphylococcus aureus* was obtained as early as after 8 hours of incubation, while for the unmodified materials, a 6 log decrease in bacterial population was obtained only after 24 hours (Figure 10 - A and B).

The *Escherichia coli* strain proved more resistant to the materials. During 24 hours of incubation, a bacteriostatic activity was demonstrated for ZnO50 and ZnO100. The materials modified with chitosan resulted in a 6 log reduction in the *E. coli* population (Figure 11. A and B).

As mentioned in the introduction, the antibacterial activity of zinc oxide nanoparticles or Zn/CS nanocomposites is widely described. The demonstrated bactericidal effect of the presented CS@ZnO materials is their additional advantage.

### Cytotoxicity assays

The cytotoxic effect is an important, previously mentioned aspect, which is a result of photocatalytic reactions occurring on the surface of semiconductors and reactive oxygen species formed in this way. In order to quantitatively determine the cytotoxic activity of the materials against human keratinocytes (HaCaT), a measurement of inhibition of cell growth using mitochondrial redox activity - MTT method - was performed. The mitochondrial oxidoreductive activity in living cells is measured as the ability to reduce the MTT dye. As a result of the activity of mitochondrial dehydrogenase, a yellow water-soluble tetrazolium salt is converted into water-insoluble crystals of formazan, the amount of which is proportional to the amount of the living cells. The crystals, dissolved in a DMSO:MeOH (1:1) solvent mixture, produce a colored solution, which is then subjected to spectrophotometric analysis on a Tecan plate reader. The results are presented as a percentage of a control or the absorbance measured for the cells which were not treated with the test materials. Cells seeded on 96-well plates at a density (1×10⁴) were treated with ZnO and CS@ZnO suspensions at a concentration of 0.5 mg/ml (the final concentration after the dilution in the cell medium was 0.25 mg/mL) and incubated for 24 h. After this time, the MTT test (Figure 12) was performed.

Only in case of unmodified ZnO50 nanoparticles, a slight cytotoxic effect at a level of ~15% was obtained, which was not reported for ZnO100 nanoparticles. The difference in ZnO50 and ZnO100 activity is most probably the result of differences in nanoparticle size. The smaller their size, the higher their penetration into the cells and the potential cytotoxic effect^{23,24}. For the CS@ZnO materials, no cytotoxic effect was observed. Surface modification of nanoparticles with a polymer is known in literature as a method to minimize their toxic activity²⁵. The lack of cytotoxic effect, leading to increased biocompatibility, is an additional advantage of the presented CS@ZnO materials.

### Literature

1. Buchalska, M.; Kras, G.; Oszajca, M.; 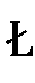 asocha, W.; Macyk, W. Journal of Photochemistry and PhotobiologyA: Chemistry 2010, 213, (2-3), 158-163.
2. Ko 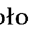 odziejczak-Radzimska, A.; Jesionowski, T. Materials 2014, 7, (4), 2833-2881.
3. Lansdown, A. B. G.; Taylor, A. International Journal of Cosmetic Science 1997, 19, (4), 167-172.
4. Cross, S. E.; Innes, B.; Roberts, M. S.; Tsuzuki, T.; Robertson, T. A.; McCormick, P. Skin Pharmacology and Physiology 2007, 20, (3), 148-154.
5. Janczyk, A.; Krakowska, E.; Stochel, G.; Macyk, W. Journal of the American Chemical Society 2006, 128, (49), 15574-15575.
6. Janczyk, A.; Wolnicka-G 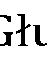 ubisz, A.; Urbanska, K.; Kisch, H.; Stochel, G.; Macyk, W. Free Radical Biology and Medicine 2008, 44, (6), 1120-1130.
7. Serpone, N.; Dondi, D.; Albini, A. Inorganica Chimica Acta 2007, 360, (3), 794-802.
8. Newman, M. D.; Stotland, M.; Ellis, J. I. Journal of the American Academy of Dermatology 2009, 61, (4), 685-692.
9. McHugh, P. J.; Knowland, J. Photochemistry and Photobiology 1997, 66, (2), 276-281.
10. Dunford, R.; Salinaro, A.; Cai, L.; Serpone, N.; Horikoshi, S.; Hidaka, H.; Knowland, J. FEBS Letters 1997, 418, (1-2), 87-90.
11. Janczyk, A.; Wolnicka-G 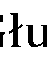 ubisz, A.; Urbanska, K.; Stochel, G.; Macyk, W. Journal of Photochemistry and Photobiology B: Biology 2008, 92, (1), 54-58.
12. Lee, W. A.; Pernodet, N.; Li, B.; Lin, C. H.; Hatchwell, E.; Rafailovich, M. H. Chemical Communications 2007, (45), 4815-4817.
13. Oberdorster, G.; Maynard, A.; Donaldson, K.; Castranova, V.; Fitzpatrick, J.; Ausman, K.; Carter, J.; Karn, B.; Kreyling, W.; Lai, D.; Olin, S.; Monteiro-Riviere, N.; Warheit, D.; Yang, H. Particle and Fibre Toxicology 2005, 2, (1), 8.
14. Kim, S.; Lim, Y. T.; Soltesz, E. G.; De Grand, A. M.; Lee, J.; Nakayama, A.; Parker, J. A.; Mihaljevic, T.; Laurence, R. G.; Dor, D. M.; Cohn, L. H.; Bawendi, M. G.; Frangioni, J. V. Nat Biotech 2004, 22, (1), 93-97.
15. Peters, K.; Unger, R.; Kirkpatrick, C. J.; Gatti, A.; Monari, E. Journal of Materials Science: Materials in Medicine 2004,15, (4), 321-325.
16. Dodd, A. C.; McKinley, A. J.; Saunders, M.; Tsuzuki, T.J Nanopart Res 2006, 8, (1), 43-51.
17. Mirhosseini, M.; Firouzabadi, F. B. International Journal of Dairy Technology 2013, 66, (2), 291-295.
18. Li, L.-H.; Deng, J.-C.; Deng, H.-R.; Liu, Z.-L.; Xin, L. Carbohydrate Research 2010, 345, (8), 994-998.
19. Vaseeharan, B.; Sivakamavalli, J.; Thaya, R. Journal of Composite Materials 2013.
20. AbdElhady, M. M. International Journal of Carbohydrate Chemistry 2012, 2012, 6.
21. Farouk, A.; Moussa, S.; Ulbricht, M.; Textor, T. International Journal of Carbohydrate Chemistry 2012, 2012, 8.
22. Sanoop, P. K.; Mahesh, K. V.; Nampoothiri, K. M.; Mangalaraja, R. V.; Ananthakumar, S. Journal of Applied Polymer Science 2012, 126, (S1), E233-E244.
23. Kong, B.; Seog, J. H.; Graham, L. M.; Lee, S. B. Nanomedicine 2011, 6, (5), 929-941.
24. Shang, L.; Nienhaus, K.; Nienhaus, G. Journal of Nanobiotechnology 2014, 12, (1), 5.
25. Sun, L.; Huang, C.; Gong, T.; Zhou, S. Materials Science and Engineering: C 2010, 30, (4), 583-589.

## Claims

1. A process for preparation of a colloid containing a composite, **characterized in that**:
a) a suspension of grains of zinc oxide in a solution of chitosan is obtained,
b) the resulting suspension is mixed with a solution of tripolyphosphate, and a reaction of ionotropic crosslinking is conducted,
c) the resulting mixture is treated with ultrasound, and the colloid containing the composite is obtained.

2. The process of claim 1, **characterized in that** in step a) the grains of zinc oxide have a size not greater than 100 nm.

3. The process of claim 1, **characterized in that** the suspension obtained in step a) comprises equal concentrations of zinc oxide and chitosan.

4. The process of claim 1, **characterized in that** the mixture obtained in step b) comprises tripolyphosphate at a concentration twice as high as the concentration of chitosan.

5. A colloid containing a composite comprising aggregates consisting of grains of zinc oxide trapped in chitosan ionotropically crosslinked with tripolyphosphate, wherein the aggregates have a size not greater than 100 micrometers.

## Patentansprüche

1. Verfahren zur Präparation eines Kolloid-enthaltenden Komposit, **dadurch gekennzeichnet, dass**
a) eine Suspension von Körnern von Zinkoxid in einer Lösung von Chitosan erhalten wird,
b) die sich ergebende Suspension mit einer Lösung von Tripolyphosphat gemischt wird, und eine Reaktion von ionotropem Kreuzvernetzen durchgeführt wird,
c) dass sich ergebende Gemisch wird mit Ultraschall behandelt, und das Kolloid enthaltend das Komposit wird erhalten.

2. Verfahren nach Anspruch 1, **dadurch gekennzeichnet, dass** in Schritt a) die Körner des Zinkoxids eine Größe von nicht mehr als 100 nm aufweisen.

3. Verfahren nach Anspruch 1, **dadurch gekennzeichnet, dass** die in Schritt a) erhaltende Suspension gleiche Konzentrationen an Zinkoxid und Chitosan umfasst.

4. Verfahren nach Anspruch 1, **dadurch gekennzeichnet, dass** das in Schritt b) erhaltende Gemisch Tripolyphosphat bei einer Konzentration umfasst, die zweifach höher ist als die Konzentration von Chitosan.

5. Kolloid, enthaltend ein Komposit umfassend Aggregate bestehend aus Körnern von Zinkoxid gefangen in Chitosan ionotropisch kreuzvernetzt mit Tripolyphosphat, wobei die Aggregate eine Größe von nicht mehr als 100 Mikrometern aufweisen.

## Revendications

1. Procédé de préparation d'un colloïde contenant un composite, **caractérisé en ce que** :
a) une suspension de grains d'oxyde de zinc dans une solution de chitosane est obtenue,
b) la suspension résultante est mélangée avec une solution de tripolyphosphate, et une réaction de réticulation ionotrope est réalisée,
c) le mélange résultant est traité avec des ultrasons, et le colloïde contenant le composite est obtenu.

2. Procédé selon la revendication 1, **caractérisé en ce que** dans l'étape a) les grains d'oxyde de zinc ont une taille non supérieure à 100 nm.

3. Procédé selon la revendication 1, **caractérisé en ce que** la suspension obtenue dans l'étape a) comprend des concentrations égales d'oxyde de zinc et de chitosane.

4. Procédé selon la revendication 1, **caractérisé en ce que** le mélange obtenu dans l'étape b) comprend du tripolyphosphate à une concentration deux fois plus élevée que la concentration de chitosane.

5. Colloïde contenant un composite comprenant des agrégats constitués de grains d'oxyde de zinc piégés dans du chitosane réticulé de manière ionotrope avec du tripolyphosphate, dans lequel les agrégats ont une taille non supérieure à 100 micromètres.
